# EUROPÄISCHE PATENTSCHRIFT

(11) **EP 3 530 229 B1**
(45) Veröffentlichungstag und Bekanntmachung des Hinweises auf die Patenterteilung: **03.06.2020**
(21) Anmeldenummer: 19158684.1
(22) Anmeldetag: 21.02.2019
(51) Int. Cl.: A61B 90/57, A61B 17/34, A61B 17/00, A61B 17/50

(54) **VORRICHTUNG ZUR SIMULTANEN FIXIERUNG VON MEDIZINISCHEN INSTRUMENTEN UND ENTSPRECHENDES SYSTEM**
DEVICE FOR THE SIMULTANEOUS FIXING OF MEDICAL INSTRUMENTS AND CORRESPONDING SYSTEM
DISPOSITIF DE FIXATION SIMULTANÉE DES INSTRUMENTS MÉDICAUX ET SYSTÈME CORRESPONDANT

(30) Priorität: 22.02.2018 DE 102018103968
(43) Veröffentlichungstag der Anmeldung: 28.08.2019
(73) Patentinhaber: Karl Storz SE & Co. KG, 78532 Tuttlingen (DE)
(72) Erfinder: Göbel, Werner, 78532 Tuttlingen (DE); Huber, Florian, 78532 Tuttlingen (DE); Glöggler, Bernhard, 78532 Tuttlingen (DE)
(74) Vertreter: Witte, Weller & Partner Patentanwälte mbB

(56) Entgegenhaltungen:
- US-A1- 2004 243 146
- US-A1- 2008 183 191
- US-A1- 2011 118 545

## Beschreibung

Die vorliegende Erfindung betrifft eine Vorrichtung zur simultanen Fixierung von medizinischen Instrumenten sowie ein System mit einer solchen Vorrichtung.

Die Positionierung von medizinischen Instrumenten erfolgt in der klinischen Routine nach wie vor per Hand oder mit speziellen Haltearmsystemen. Dabei ist insbesondere die Positionierung eines in einem Trokar eingeführten Instruments von Interesse. Es sei dabei lediglich beispielhaft auf die Veröffentlichung US 2012/0296281 A1 verwiesen. US2008/183191 offenbart einen Instrumentenführer umfassend einen Führungskörper mit einem kugelförmigen Teil gehalten und eingeklemmt durch zwei Verriegelungsarme.

Zu den Anforderungen bei der Positionierung kann zum einen eine schnelle Möglichkeit der Positionierung in axialer Richtung, also entlang einer Längsachse, insbesondere der Endoskopachse, zählen. Ferner ist oftmals eine Rotationsmöglichkeit um eine Einstichstelle, insbesondere einem Trokareinstich, gewünscht. Der Schwenkpunkt bzw. Pivotpunkt wird dabei bevorzugt in unmittelbarer Nähe der Einstichstelle gewählt, um bei einer Verlagerung eine mechanische Belastung, insbesondere eine Zerrung, an der Einstichstelle zu vermeiden.

WO 2016/144180 A1 zeigt einen maßgeschneiderten Trokar mit einem komprimierbaren Kugelgelenk. An dem Kugelgelenk ist ein Überwurfband angebracht, welches beim Zusammenziehen des Bandes eine Kugel des Kugelgelenks komprimiert und somit eine Fixierung eines in den Trokar eingeführten Instruments erzielt. Es verbleibt jedoch u.a. der Wunsch, dass die für das Halten verwendete Vorrichtung flexibler bezüglich verschiedener Instrumente ist, insbesondere im Hinblick auf einen Durchmesser der Instrumente.

Es ist eine Aufgabe der vorliegenden Erfindung, eine Vorrichtung zur simultanen Fixierung von medizinischen Instrumenten aufzuzeigen, die eine Halterung von Instrumenten mit verschiedenen Durchmessern ermöglicht und mit einer Hand bedient werden kann. Ferner soll ein entsprechendes System mit zwei medizinischen Instrumenten aufgezeigt werden.

Gemäß einem ersten Aspekt wird die Aufgabe gelöst durch eine Vorrichtung zur simultanen Fixierung von medizinischen Instrumenten, die Vorrichtung mit einem Gelenkteil, das einen Halteabschnitt in der Form einer Kugelscheibe aufweist, wobei die Kugelscheibe einen Mittelpunkt und eine Höhe aufweist und eine Längsachse der Vorrichtung definiert, die durch den Mittelpunkt und parallel zu der Höhe verläuft, wobei die Kugelscheibe entlang der Längsachse einen durchgängigen Hohlraum aufweist, wobei der Halteabschnitt in mindestens zwei Haltesegmente unterteilt ist und wobei an dem Gelenkteil mindestens zwei Armelemente angeordnet sind, die jeweils in einem ersten Armabschnitt ausgehend von einem Befestigungspunkt am Gelenkteil sich von der Längsachse entfernen, in einem zweiten Armabschnitt sich der Längsachse annähern und mit einer Auflagefläche enden, wodurch ein Freiraum zwischen den Armelementen gebildet ist, die Vorrichtung ferner mit einem Spannelement, welches dafür ausgebildet ist, die Haltesegmente zusammenzudrücken, wobei bei einem Zusammendrücken der Haltesegmente sich die Auflageflächen auf die Längsachse zu bewegen.

Die Unterteilung in Haltesegmente erfolgt so, dass die Segmentierung bei Betrachtung in einer Ebene senkrecht zur Längsachse erkennbar ist. Das heißt, eine Trennebene zwischen zwei Haltesegmenten verläuft nicht senkrecht zur Längsachse. Bei vorteilhaften Ausgestaltungen steht die Trennebene zur Längsachse in einen Winkel von weniger als 45°, bevorzugt weniger als 30°, besonders bevorzugt weniger als 15° und insbesondere weniger als 5°. Die Trennebene zwischen zwei Haltesegmenten ist bei einer vorteilhaften Ausgestaltung eine ebene Fläche, die sich von der Längsachse ausgehend nach außen erstreckt. Mathematisch formuliert beinhaltet die Trennebene dann den Richtungsvektor der Längsachse.

Dabei ist es eine Besonderheit der Vorrichtung, dass durch den Halteabschnitt ein Instrument geführt werden kann und der Halteabschnitt im Zusammenspiel mit dem Spannelement dafür ausgebildet ist, das Instrument zu führen. Dabei ist es bevorzugt, dass durch eine Dosierung der durch das Spannelement aufgebrachten Kraft eingestellt werden kann, wie eng die Führung des Instruments erfolgen soll. Die Trennebene ist bevorzugt derart ausgestaltet, dass zwischen zwei Haltesegmenten ein definierter Abstand vorhanden ist, wenn sie konzentrisch zueinander angeordnet sind. Der Abstand zwischen den Haltesegmenten stellt die Funktionalität des Klemmmechanismus sicher. Die Trennebene kann dann auch als Trennvolumen verstanden werden, also wie eine Ebene mit einer Stärke.

Eine weitere Besonderheit der Vorrichtung ist, dass die Kraft, die mittels des Spannelements auf den Halteabschnitt ausgeübt wird, über die Armelemente auf die Auflageflächen übertragen wird und ein zwischen den Auflageflächen befindliches Instrument festgeklemmt wird. Umgekehrt kann durch Lösen des Spannelements ein durch die Auflagefläche ausgeübter Druck verringert oder aufgehoben werden und damit die Klemmung des Instruments verringert werden oder aufgehoben werden. Da somit die Halterung und/oder die Fixierung der Instrumente an unterschiedlichen Stellen erfolgt, die zudem die Möglichkeit bieten, unterschiedliche Durchmesser der Instrumente zu berücksichtigen, können unterschiedliche Instrumentenkombinationen verwendet werden, ohne eine einfache Bedienbarkeit aufzugeben.

Unabhängig von der Anzahl der Halteelemente und der Armelemente ist es im Hinblick auf eine Austauschbarkeit der einzelnen Elemente bevorzugt, dass jedes Halteelement bezogen auf die Längsachse denselben Winkel um die Längsachse beschreibt. Insbesondere ist es dabei bevorzugt, dass die Summe der Winkel aller Haltesegmente zumindest ungefähr 360° beträgt. Werden vier Haltesegmente verwendet, so beschreibt jedes Haltesegment bevorzugt einen Winkel von 90° um die Längsachse. Werden drei Haltesegmente verwendet, so beschreibt bevorzugt jedes Halteelement einen Winkel von 120° um die Längsachse. Diese Ausgestaltung ermöglicht ein gutes Kontrollieren des Gelenkteils durch Greifen von einem der Armelemente. Werden zwei Haltesegmente verwendet, so beschreibt bevorzugt jedes Halteelement einen Winkel von 180° um die Längsachse.

Einer der Vorteile der offenbarten Technik ist es, dass die Halterung unabhängig vom verwendeten Trokar ermöglicht wird. Ferner werden medizinische Instrumente unterschiedlicher Bauart fixiert oder gehalten.

Dadurch ist die Aufgabe vollständig gelöst.

Bei einer vorteilhaften Ausgestaltung weist das Gelenkteil einen Flansch auf, der sich ausgehend von dem Halteabschnitt entlang der Längsachse erstreckt und eine Fortsetzung des Hohlraums bildet, wobei der Flansch in mindestens zwei Flanschsegmente unterteilt ist, die insbesondere jeweils mit genau einem Haltesegment verbunden sind.

Der Flansch bietet unter anderem eine vorteilhafte Möglichkeit für die Befestigung der Armelemente. Da mittels des Flansches die eigentliche Oberfläche der Kugelscheibe freigehalten wird, kann der Halteabschnitt besonders frei verlagert werden. Der Flansch kann auch bewirken, dass ein vom Halteabschnitt gehaltenes erstes Instrument, insbesondere eines Trokars, zumindest locker entlang der Längsachse gehalten wird. Ferner kann der Flansch dabei helfen, dass eine seitliche Druckbelastung auf die medizinischen Instrumente, insbesondere das erste Instrument, zu einer möglichst geringen Verlagerung von der Längsachse wegführt. Über die Länge des Flanschs kann auch eine Kontaktfläche zwischen dem Gelenkteil und dem ersten Instrument gewählt werden, so dass ein Druck auf den Halteabschnitt, und damit auf den Flansch, bei einer großen Kontaktfläche zu einer schnellen Fixierung führt und bei einer kleinen Fläche zu einer fein dosierbaren Fixierung führt. Der Flansch bzw. die Flanschsegmente sind bei vorteilhaften Ausgestaltungen einstückig mit dem Halteabschnitt bzw. den Haltesegmenten ausgebildet. Bei anderen vorteilhaften Ausgestaltungen ist der Flansch bzw. sind die Haltesegmente lösbar am Halteabschnitt bzw. den Haltesegmenten angeordnet. Dies kann beispielsweise durch eine Schraub- oder Steckverbindung erfolgen. Ein bestehender Flansch kann dann gegen einen Flansch mit anderen Dimensionen, insbesondere einem anderen Durchmesser, ausgetauscht werden. Die Anzahl der Flanschsegmente muss nicht mit der Anzahl der Haltesegmente übereinstimmen. So kann es je nach Anwendung beispielsweise ausreichend sein, wenn bei drei Haltesegmenten nur zwei Flanschsegmente vorhanden sind oder wenn bei vier Haltesegmenten zwei oder drei Flanschsegmente vorhanden sind. Grundsätzlich können auch mehr Flanschsegmente als Haltesegmente vorhanden sein, die z.B. aneinandergesteckt werden. Nach derzeitiger Einschätzung scheint dies jedoch die Handhabung zu erschweren. Da der Flansch eine Fortsetzung des Hohlraums bildet, kann das erste Instrument weiterhin durch das gesamte Gelenkteil geführt werden, d.h. sowohl durch den Halteabschnitt als auch durch den Flansch. Zur Segmentierung des Flanschs gelten sinngemäß dieselben Ausführungen wie zur Segmentierung des Gelenkteils. Insbesondere ist die Segmentierung des Flanschs gleich gewählt wie die Segmentierung des Gelenkteils.

Bei einer weiteren vorteilhaften Ausgestaltung weist die Kugelscheibe einen Durchmesser auf, und das Verhältnis von der Höhe zu dem Durchmesser liegt zwischen 0,35 und 0,99, bevorzugt zwischen 0,47 und 0,93, besonders bevorzugt zwischen 0,58 und 0,87 und insbesondere zwischen 0,7 und 0,8.

Dieser Ausgestaltung liegt die Überlegung zugrunde, dass bei einer flachen Kugelscheibe, d.h. das Verhältnis von Höhe zu Durchmesser ist eher klein, ein großer Hohlraum im Gelenkteil realisiert werden kann, so dass Instrumente mit einem großen Durchmesser relativ zum Durchmesser des Gelenkteils fixiert werden können. Bei einer flachen Kugelscheibe ist jedoch der Schwenkbereich des Gelenkteils relativ zum Spannelement eher gering. Wenn man die Kugelscheibe sehr hoch wählt, d.h. das Verhältnis von Höhe zu Durchmesser ist eher groß und der Halteabschnitt nähert sich zumindest ungefähr einer Kugelform an, so ergibt sich ein besonders großer Schwenkbereich. In diesem Fall wird der verfügbare Durchmesser des Hohlraums im Gelenkteil jedoch relativ klein im Vergleich zum Gesamtdurchmesser des Gelenkteils, so dass auch nur ein verhältnismäßig schmales erstes Instrument durch das Gelenkteil geführt werden kann.

Bei einer weiteren vorteilhaften Ausgestaltung weist die Vorrichtung ferner einen Spannring auf, der von den Auflageflächen gehalten ist, wobei bei einem Zusammendrücken der Haltesegmente die Auflageflächen gegen den Spannring drücken.

Diese Ausgestaltung gibt eine zusätzliche Möglichkeit, das Klemmen des medizinischen Instruments zu steuern.

Bei einer weiteren vorteilhaften Ausgestaltung sind die Armelemente verschwenkbar am Spannring angeordnet.

Diese Ausgestaltung kann zum einen eine vereinfachte Handhabung bieten und unterstützen, dass die Haltesegmente geführt auf die Längsachse zu und von der Längsachse weg bewegt werden können. Da die Armelemente verschwenkbar am Spannring angeordnet sind, lassen sich diese besonders einfach zum Halteabschnitt des Gelenkteils zusammensetzen. Zum anderen ergibt sich nun ein Gegenlager, wenn die Auflageflächen der Armelemente gegen den Spannring drücken. Bei einer vorteilhaften Ausgestaltung sind die Armelemente zudem lösbar am Spannring angeordnet. Die Armelemente können dann z.B. zum Sterilisieren vom Spannring gelöst werden. Wenn der Spannring aus einem flexiblen Material gefertigt ist, können die Armelemente insbesondere über eine Schnappverbindung am Spannring befestigt werden.

Bei einer weiteren vorteilhaften Ausgestaltung der Erfindung sind die Auflageflächen gebogen, um der Form des Spannrings zu folgen.

Diese Ausgestaltung ermöglicht es, dass der durch die Auflageflächen ausgeübte Druck auf eine größere Fläche des Spannrings verteilt wird. Daraus kann sich insbesondere eine gleichmäßigere Kraftverteilung entlang des Umfangs des Spannrings ergeben. Bevorzugt entspricht der innere Radius, bezogen auf die Längsachse, der Auflageflächen dem äußeren Radius des Spannrings in dem Bereich, an dem die Auflageflächen anliegen.

Bei einer weiteren vorteilhaften Ausgestaltung weist der Spannring für jede Auflagefläche eine Aufnehmung auf, in die eine jeweilige Auflagefläche lösbar eingehängt werden kann, so dass das jeweilige Armelement um die jeweilige Auflagefläche als Schwenkpunkt relativ zur Längsachse verschwenkt werden kann.

Diese Ausgestaltung ermöglicht eine gute Handhabbarkeit der Vorrichtung. Durch das Einhängen der Armelemente kann einerseits eine gute Vorpositionierung der Armelemente und damit der Haltesegmente erfolgen. Andererseits sind die Armelemente aber lösbar am Spannring eingehängt, so dass eine gute Autoklavierbarkeit der Armelemente gewährleistet bleibt.

Bei einer weiteren vorteilhaften Ausgestaltung stehen zwei benachbarte Haltesegmente über eine Nut-Feder-Verbindung in Eingriff miteinander.

Diese Ausgestaltung ermöglicht es, dass die Haltesegmente bereits dann eine definierte Position zueinander einnehmen können, selbst wenn die Haltesegmente noch nicht an ihren Seitenflächen aneinander liegen. Ferner hilft die Nut-Feder-Verbindung dabei, dass die Halteelemente auch im entspannten Zustand des Spannelements ihre relative Position zueinander zumindest ungefähr beibehalten. Bei einer bevorzugten Ausgestaltung ist die Nut in eine der beiden Seitenflächen eines Haltesegments eingebracht und mündet in den Hohlraum. Dabei ist die Nut bevorzugt senkrecht zur Längsachse ausgerichtet. Als die Seitenflächen sind jene Flächen eines Haltesegments zu verstehen, mit denen ein Haltesegment an das benachbarte Haltesegment angrenzt.

Bei einer weiteren vorteilhaften Ausgestaltung weist der Halteabschnitt genau drei Haltesegmente auf und ist an jedem Haltesegment genau ein Armelement angeordnet.

Diese Ausgestaltung ermöglicht einerseits eine gute Handhabbarkeit, da sich drei Haltesegmente gut zusammensetzen lassen. Zudem bietet die Aufteilung in drei Haltesegmente eine gute Kraftverteilung auf ein gehaltenes zweites Instrument. Soll die Handhabbarkeit verbessert werden, so ist es bevorzugt, genau zwei Haltesegmente zu verwenden. Soll die Kraftverteilung und das Anliegen der einzelnen Armelemente am zweiten medizinischen Instrument verbessert werden, so sind vier, fünf, sechs oder mehr Haltesegmente bevorzugt. Der Aspekt, dass an jedem Haltesegment genau ein Armelement angeordnet ist, ist zwar unabhängig von den genau drei Haltesegmenten, doch wird es im Hinblick auf die Austauschbarkeit der Haltesegmente als vorteilhaft angesehen, dass jedes der drei Haltesegmente genau ein Armelement aufweist, so dass auch genau drei Armelemente vorhanden sind.

Bei einer weiteren vorteilhaften Ausgestaltung der Erfindung sind die Armelemente bezogen auf einen Winkel um die Längsachse um mindestens 45°, bevorzugt um mindestens 60°, besonders bevorzugt um mindestens 90° und insbesondere um mindestens 120° voneinander beabstandet.

Diese Ausgestaltung ermöglicht eine gute Zugänglichkeit zur Längsachse der Vorrichtung im Bereich der Armelemente, selbst wenn viele Armelemente verwendet werden. Gerade wenn es sich bei dem ersten Instrument um ein Instrument mit einem oder mehreren Anschlüssen handelt, sind die Anschlüsse des ersten Instruments bei dieser Ausgestaltung immer noch gut erreichbar. Bei einer bevorzugten Ausgestaltung, die drei Haltesegmente aufweist, ist jedes Armelement zum benachbarten Armelement um 120° voneinander beabstandet.

Bei einer weiteren vorteilhaften Ausgestaltung weist jedes Armelement einen dritten Armabschnitt auf, der zwischen dem ersten Armabschnitt und dem zweiten Armabschnitt angeordnet ist und sich insbesondere zumindest ungefähr parallel zu der Längsachse erstreckt.

Diese Ausgestaltung ermöglicht die Verwendung von sehr verschiedenen Trokarformen. Durch eine solche Ausbildung der Armelemente, insbesondere der Länge der zweiten Armabschnitte, können auch Trokare mit großen Köpfen verwendet werden. Außerdem kann diese Ausgestaltung ein bequemes Greifen des Armelements und dadurch ein gut kontrollierbares Verlagern des Gelenkteils ermöglichen.

Bei einer weiteren vorteilhaften Ausgestaltung weist das Spannelement zwei Spannhebel auf, die mit einem Scharnier verbunden sind, wobei jeder Spannhebel einen Druckbereich aufweist, der dafür ausgebildet ist, das Gelenkteil zu halten und gegen den Halteabschnitt zu drücken, wenn die Spannhebel zusammengedrückt werden, wobei die Spannhebel insbesondere mit einer Spannschraube verbunden sind, die dafür ausgebildet ist, die Spannhebel zusammenzudrücken.

Diese Ausgestaltung bietet eine gute Herstellbarkeit und kann zuverlässig sterilisiert werden. Bei einer bevorzugten Ausgestaltung ist der Druckbereich als konische Auflagefläche ausgebildet, deren Radius insbesondere zumindest ungefähr dem Radius der Kugelscheibe entspricht. Bei anderen bevorzugten Ausführungsformen ist der Druckbereich jedes Spannhebels aus zwei linienförmigen Auflagen gebildet, wobei die linienförmigen Auflagen bevorzugt zumindest ungefähr parallel zueinander verlaufen. Eine solche Ausgestaltung ist beispielsweise dadurch zu erzielen, dass der Radius der konischen Auflagefläche kleiner ist als der Radius der Kugelscheibe. Das Gelenkteil liegt dann nur an Begrenzungen der Auflagefläche auf, so dass die Auflagefläche selbst weggelassen werden kann.

Bei einer weiteren vorteilhaften Ausgestaltung weist der Spannring ein ringförmiges Basiselement und mindestens zwei Fortsätze auf, die relativ zum Basiselement auf die Längsachse zu verlagerbar sind und wobei der Spannring an den Fortsätzen gehalten ist.

Diese Ausgestaltung ermöglicht eine besonders gute Kontrolle über die Klemmwirkung des Spannrings. Die Fortsätze sind nicht direkt miteinander verbunden, sondern lediglich gemeinsam an dem Basiselement angeordnet. Durch die geometrische Ausgestaltung der Fortsätze kann bestimmt werden, wie stark sich die Fortsätze unter der Krafteinwirkung durch die Auflageflächen auf die Längsachse zu verlagern. Dabei gilt im Allgemeinen, dass je weiter sich die Fortsätze in Richtung der Längsachse verbiegen können, desto größer ist der Bereich der möglichen Durchmesser des zweiten Instruments, die von dem Spannring zuverlässig gehalten werden können. Wird der Spannring über die Fortsätze von den Auflageflächen mit einem Druck beaufschlagt, so bleibt das Basiselement bevorzugt im Wesentlichen formstabil, während sich die Fortsätze durch ein Verbiegen mit ihrem freien Ende auf die Längsachse zu bewegen.

Bei einer weiteren vorteilhaften Ausgestaltung ist in den Halteabschnitt ein elastisches Halteelement eingesetzt, das dafür ausgebildet ist, ein erstes Instrument der medizinischen Instrumente entlang der Längsachse aufzunehmen.

Bei dieser Ausgestaltung ermöglicht das elastische Halteelement Unterschiede im Durchmesser zwischen einem ersten Innendurchmesser des Gelenkteils, ggf. in Verbindung mit einem Flansch, und einem Außendurchmesser des ersten medizinischen Instruments zu kompensieren. In Abhängigkeit von der gewählten Stärke des Halteelements können dann auch erste Instrumente in dem Gelenkteil geführt werden, deren Außendurchmesser erheblich kleiner ist als der Innendurchmesser des Gelenkteils.

Bei einer weiteren vorteilhaften Ausgestaltung ist der Halteabschnitt dafür ausgebildet, ein erstes Instrument der medizinischen Instrumente zu führen, und der Spannring ist dafür ausgebildet, ein zweites Instrument der medizinischen Instrumente zu halten.

Da die Instrumente an verschiedenen Stellen gehalten werden, ist eine besonders gute individuelle Positionierung und Fixierung möglich.

Bei einer weiteren vorteilhaften Ausgestaltung ist der Halteabschnitt dafür ausgebildet, einen Trokar zu führen und ist der Spannring dafür ausgebildet, ein Endoskop zu halten, das in den Trokar eingeführt ist.

Diese Ausgestaltung ist auf einen typischen Anwendungsfall ausgerichtet. Dafür liegt der Innendurchmesser des Gelenkteils bevorzugt zwischen 5 und 15 Millimetern, besonders bevorzugt zwischen 7 und 13 Millimetern und insbesondere zwischen 8 und 12 Millimetern. Der Außendurchmesser des Endoskops ist zumindest geringfügig geringer als der Innendurchmesser des Trokars und ist bevorzugt kleiner als 10 Millimeter, besonders bevorzugt kleiner als 7 Millimeter und insbesondere kleiner als 5 Millimeter.

Gemäß einem weiteren Aspekt wird die Aufgabe gelöst durch eine zuvor beschriebene Vorrichtung, einem Trokar und einem Endoskop, wobei der Trokar von dem Halteabschnitt entlang der Längsachse geführt ist und das Endoskop in dem Trokar eingeführt ist und von dem Spannring entlang der Längsachse gehalten ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine Haltevorrichtung mit einem daran gekoppelten System;
- Fig. 2: eine Ausführungsform der Vorrichtung;
- Fig. 3: die Ausführungsform gemäß Fig. 2 mit einem eingesetzten elastischen Halteelement;
- Fig. 4: das Gelenkteil und die drei Armelemente der Ausführungsform gemäß Fig. 2;
- Fig. 5: ein Haltesegment mit dem entsprechenden Armelement, das sich zu dem Spannring erstreckt;
- Fig. 6: eine weitere Ausführungsform eines Spannrings;
- Fig. 7: das Spannelement gemäß der Ausführungsform aus Fig. 2;
- Fig. 8: eine Ausführungsform einer Nut-Feder-Verbindung zwischen zwei Haltesegmenten gemäß der Ausführungsform aus Fig. 2;
- Fig. 9: eine zweite Ausführungsform eines Spannrings;
- Fig. 10: das Zusammenspiel des Spannrings aus Fig. 9 mit einer zweiten Ausführungsform von Armelementen;
- Fig. 11: eine Darstellung der Kugelscheibe;
- Fig. 12: eine dritte Ausführungsform eines Spannrings;
- Fig. 13: eine dritte Ausführungsform von Armelementen mit einer geänderten Auflagefläche; und
- Fig. 14: das Zusammenspiel des Spannrings aus Fig. 12 mit der Ausführungsform von Armelementen aus Fig. 13.

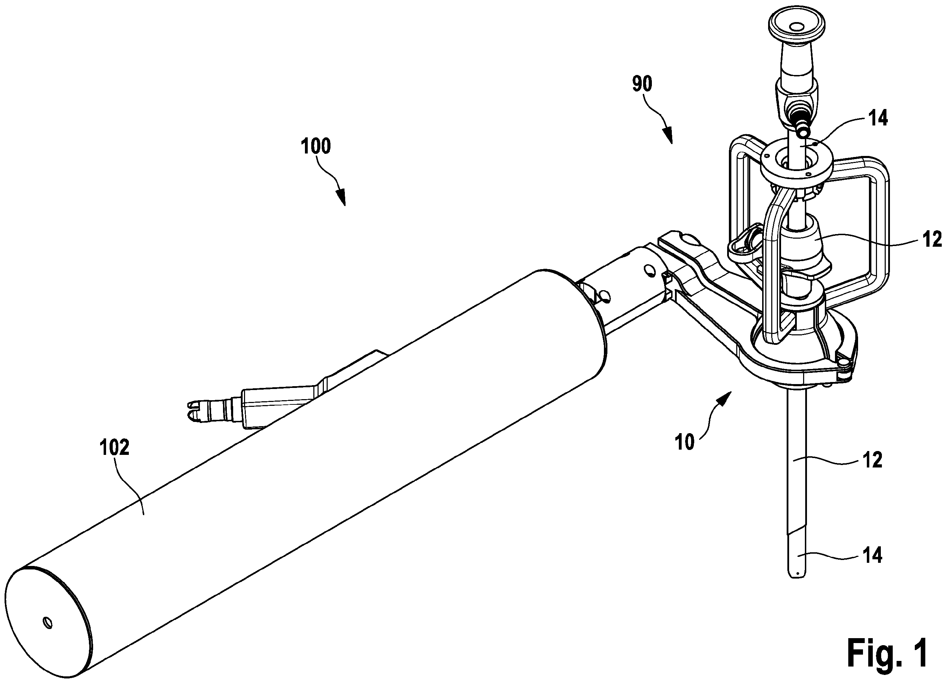

Fig. 1 zeigt einen Apparat 100, der eine Zugvorrichtung 102 aufweist, an den eine Vorrichtung 10 gekoppelt ist. Die Vorrichtung 10 fixiert ein erstes medizinisches Instrument 12, hier ein Trokar, und ein zweites medizinisches Instrument 14, hier ein Endoskop. Die Vorrichtung 10, das erste medizinische Instrument 12 und das zweite medizinische Instrument 14 bilden ein System 104. Die Vorrichtung 10 wird nun nachfolgend genauer erläutert.

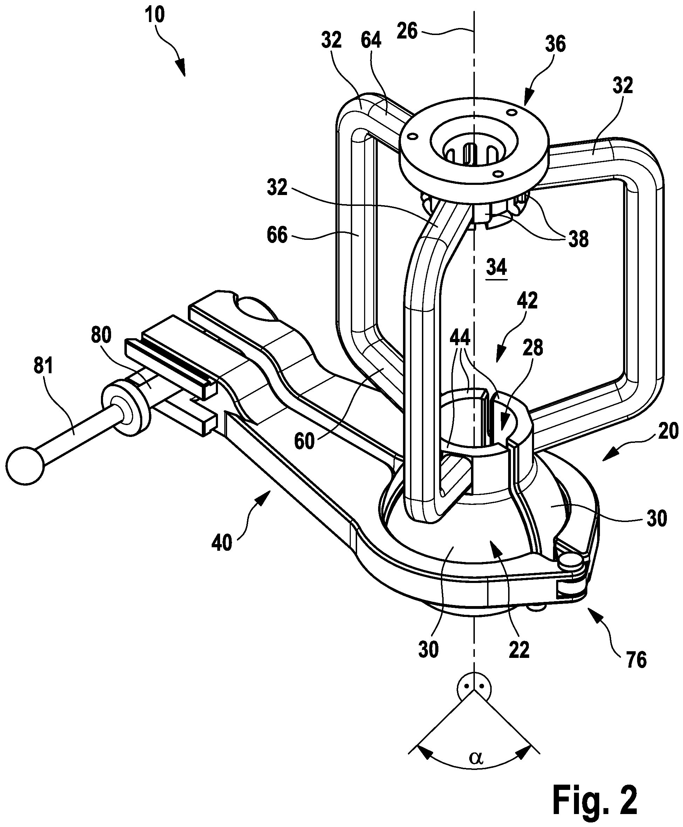

Fig. 2 zeigt die Vorrichtung 10 aus Fig. 1. Die Vorrichtung 10 weist ein Gelenkteil 20 auf, das einen Halteabschnitt 22 in Form einer Kugelscheibe 24 aufweist. Die Form der Kugelscheibe 24 wird im weiteren Verlauf noch näher erläutert. Dabei wird auch der Verlauf einer Längsachse 26 der Vorrichtung 10 erläutert.

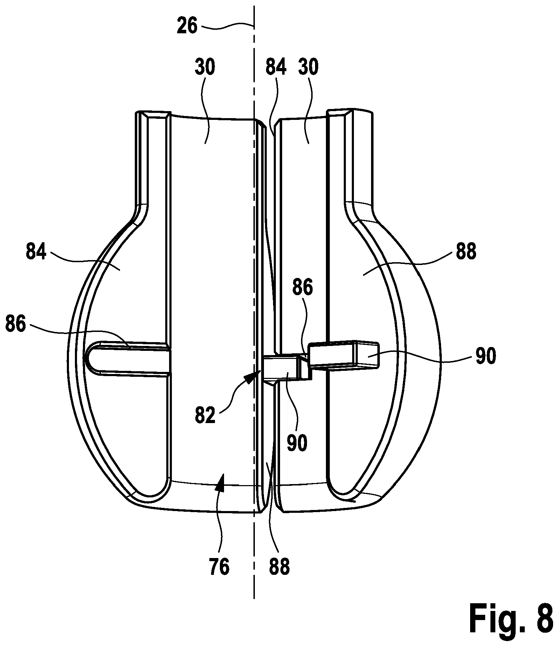

Das Gelenkteil 20 weist entlang der Längsachse 26 einen durchgängigen Hohlraum 28 auf, siehe hierzu auch die geöffnete Darstellung in Fig. 8. Der Halteabschnitt 22 ist in einer Ebene senkrecht zur Längsachse 26 in mindestens zwei Haltesegmente 30 unterteilt, bei der hier gezeigten Ausführungsform handelt es sich um drei Haltesegmente 30.

An dem Gelenkteil 20 sind mindestens zwei Armelemente 32 angeordnet, wobei die gezeigte Ausführungsform drei Armelemente 32 aufweist. Durch die Form der Armelemente 32 ist ein Freiraum 34 zwischen den Armelementen 32 gebildet.

Die Vorrichtung 10 weist ferner einen Spannring 36 auf, der von Auflageflächen 38 gehalten ist. Die Vorrichtung 10 weist zudem ein Spannelement 40 auf, das dafür ausgebildet ist, die Haltesegmente 30 zusammenzudrücken, wobei bei einem Zusammendrücken der Haltesegmente 30 die Auflageflächen 38 gegen den Spannring 36 drücken.

Das Gelenkteil 20 weist einen Flansch 42 auf, der sich ausgehend von dem Halteabschnitt 22 entlang der Längsachse 26 erstreckt und eine Fortsetzung des Hohlraums 28 bildet, wobei der Flansch 42 senkrecht zur Längsachse 26 in mindestens zwei Flanschsegmente 44 unterteilt ist. Bei dieser Ausführungsform ist der Flansch 42 in drei Flanschsegmente 44 unterteilt. Zudem ist bei dieser Ausführungsform jedes Flanschsegment 44 mit genau einem Haltesegment 30 verbunden, indem ein Haltesegment 30 und das entsprechende Flanschsegment 44 einstückig ausgebildet sind. Die Armelemente 32 sind bezogen auf einen Winkel α um die Längsachse 26 um 120° voneinander beabstandet und einstückig mit dem jeweiligen Flanschsegment (44) ausgebildet.

Der Halteabschnitt 22 ist dafür ausgebildet, das erste Instrument 12 zu führen, und der Spannring 36 ist dafür ausgebildet, das zweite Instrument 14 zu halten. Konkret ist bei dieser Ausführungsform der Halteabschnitt 22 dafür ausgebildet, einen Trokar zu halten, und der Spannring 36 ist dafür ausgebildet, ein Endoskop zu halten, das in den Trokar eingeführt ist.

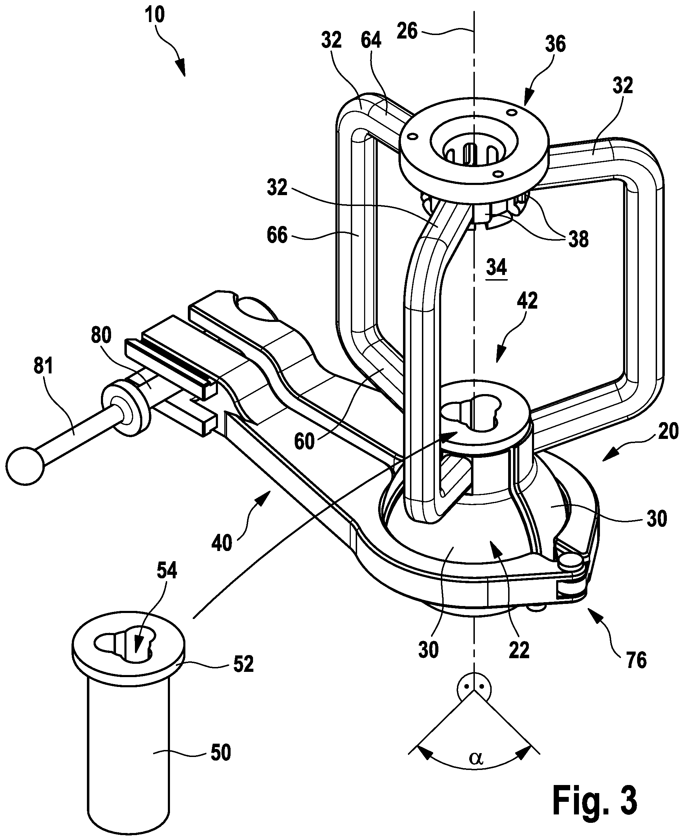

Fig. 3 zeigt die in Fig. 2 dargestellte Vorrichtung 10, wobei nun ein elastisches Halteelement 50 in den Halteabschnitt 22 eingesetzt ist, das dafür ausgebildet ist, das erste Instrument 12 entlang der Längsachse 26 aufzunehmen. Zur Verdeutlichung der Form des Halteelements 50 ist dies noch einmal außerhalb der Vorrichtung 10 dargestellt. Es ist zu erkennen, dass das Halteelement 50 eine im Wesentlichen zylindrische Form aufweist und an seinem oberen Ende mit einem Wulst 52 abschließt. Eine Ausnehmung 54 innerhalb des Halteelements 50 hat hier die Form von vier zusammengesetzten Kreisen, wobei ein Mittelpunkt eines großen ersten Kreises mit der Längsachse 26 zusammenfällt und drei weitere kleinere Kreise von der Längsachse 26 versetzt und um einen Winkel von 120° um die Längsachse beabstandet angeordnet sind.

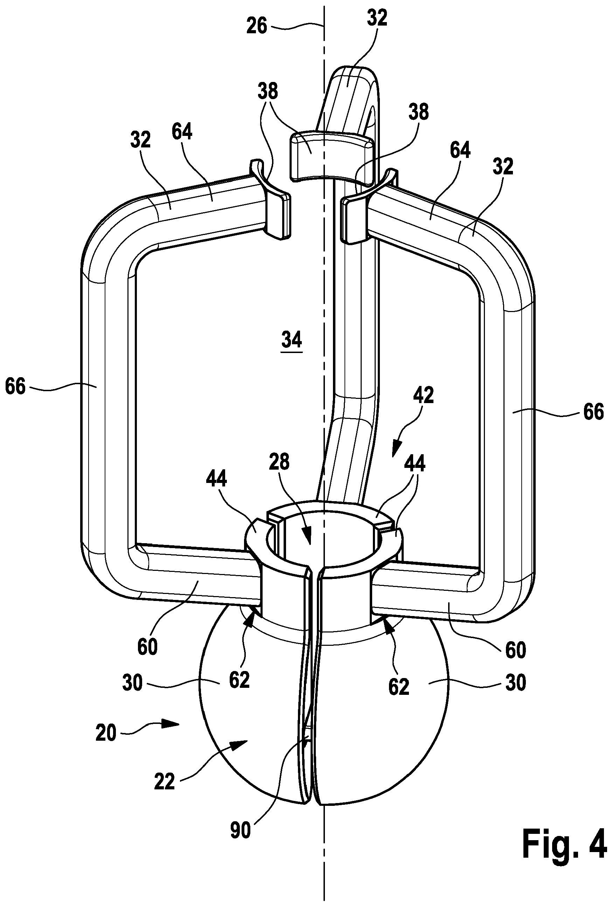

Fig. 4 zeigt das Gelenkteil 20 mit drei Armelementen 32 gemäß der Ausführungsform aus Fig. 2. Es ist zu erkennen, dass die Auflageflächen 38 der Armelemente 32 gebogen sind, um der Form des hier nicht gezeigten Spannrings 36 zu folgen.

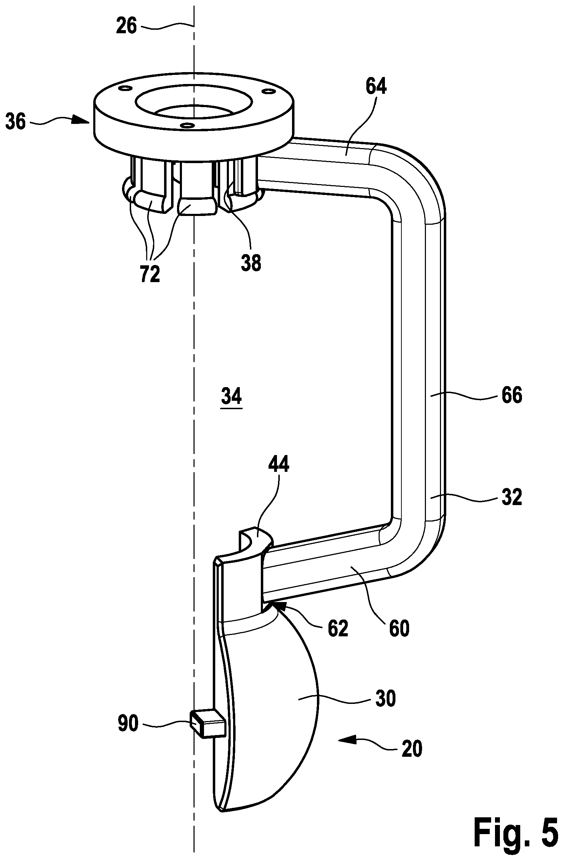

Fig. 5 zeigt ein Haltesegment 30, ein Armelement 32 und den Spannring 36. Es ist dargestellt, dass das Armelement 32, wie auch die anderen Armelemente 32, einen ersten Armabschnitt 60 ausgehend von einem Befestigungspunkt am Gelenkteil 20 aufweist, der sich von der Längsachse 26 entfernt. Am anderen Ende des Armelements 32 ist ein zweiter Armabschnitt 64 angeordnet, der sich der Längsachse 26 annähert. Der zweite Armabschnitt 64 und damit das Armelement 32 enden in der Auflagefläche 38. Das Armelement 32 weist ferner einen dritten Armabschnitt 66 auf, der zwischen dem ersten Armabschnitt 60 und dem zweiten Armabschnitt 64 angeordnet ist. Der dritte Armabschnitt 66 erstreckt sich insbesondere, wie hier gezeigt, zumindest ungefähr parallel zu der Längsachse 26.

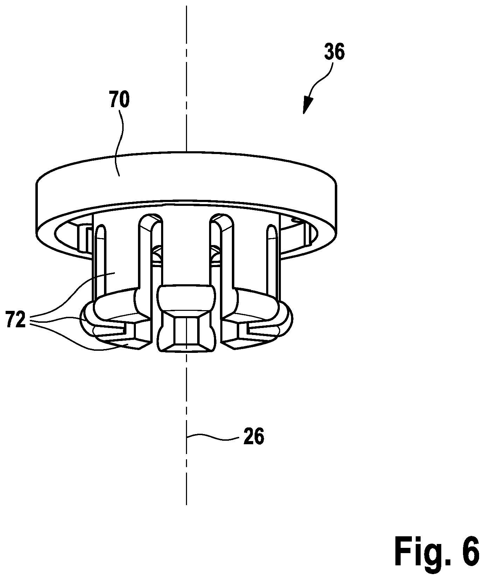

Fig. 6 zeigt den Spannring 36 gemäß der Ausführungsform aus Fig. 2. Der Spannring 36 weist ein ringförmiges Basiselement 70 und mindestens zwei Fortsätze 72 auf, wobei bei dieser Ausführungsform acht Fortsätze 72 gezeigt sind. Die Fortsätze 72 sind relativ zum Basiselement 70 auf die Längsachse 26 zu verlagerbar. Dabei bewegen sich insbesondere die freien Enden der Fortsätze 72 auf die Längsachse 26 zu. Der Spannring 36 wird von den Auflageflächen 38 an den Fortsätzen 72 gehalten.

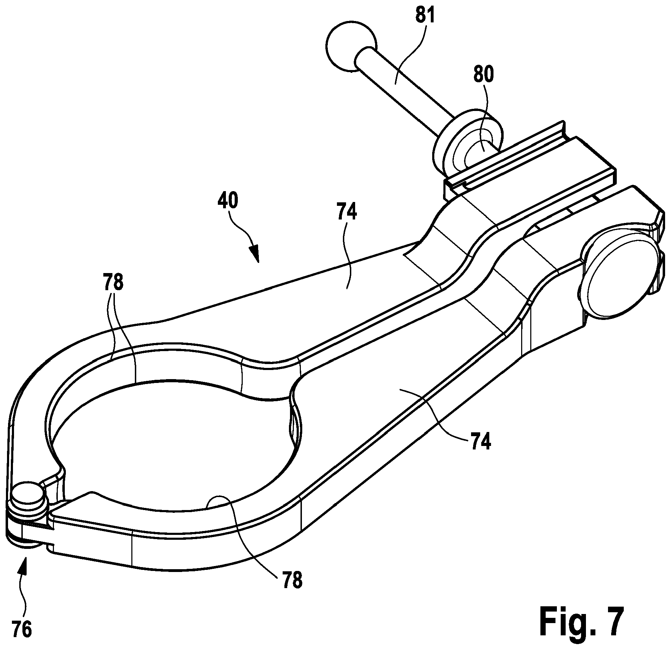

In Fig. 7 ist das Spannelement 40 aus der Ausführungsform aus Fig. 2 gezeigt. Das Spannelement 40 weist zwei Spannhebel 74 auf, die mit einem Scharnier 76 verbunden sind. Jeder Spannhebel 74 weist einen Druckbereich 78 auf, der dafür ausgebildet ist, das Gelenkteil 20 zu halten. Der Druckbereich 78 ist ferner dafür ausgebildet, gegen den Halteabschnitt 22 zu drücken, wenn die Spannhebel 74 zusammengedrückt werden. Bei dieser Ausführungsform sind die Spannhebel mit einer Spannschraube 80 verbunden, die dafür ausgebildet ist, die Spannhebel 74 zusammenzudrücken. Die Spannschraube 80 weist hier einen Fortsatz 81 aus, mit dem die Vorrichtung 10 an die Zugvorrichtung 102 gekoppelt wird.

Fig. 8 zeigt, wie bei der Ausführungsform aus Fig. 2 zwei benachbarte Haltesegmente 30 über eine Nut-Feder-Verbindung 82 in Eingriff miteinander stehen. Hierzu weisen die beiden Haltesegmente 30 an ihrer jeweils ersten Seitenfläche 84 eine Nut 86 und an ihrer jeweils zweiten Seitenfläche 88 jeweils eine Feder 90 auf. Die Nuten 86 münden bevorzugt in den Hohlraum 28. Bevorzugt sind die Nuten 86 ungefähr in der Mitte des jeweiligen Haltesegments 30 angeordnet, bezogen auf die Längsachse 26, und durchdringen nicht die äußere Oberfläche des jeweiligen Haltesegments 30.

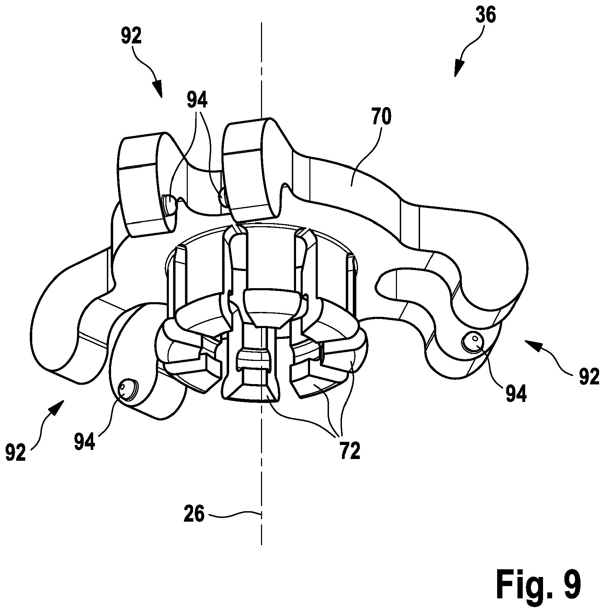

Fig. 9 zeigt eine dritte Ausführungsform eines Spannrings 36. Der Spannring 36 weist ein ringförmiges Basiselement 70 und acht Fortsätze 72 auf. Ferner sind am Spannring 36 ausgehend vom Basiselement 70 drei Halterungen 92 angebracht, die senkrecht zur Längsachse 26 hervorstehen. Jede der Halterungen 92 weist zwei Vorsprünge 94 auf, in die Armelemente 32 mit ihrem zweiten Armabschnitt 64 eingehängt werden können.

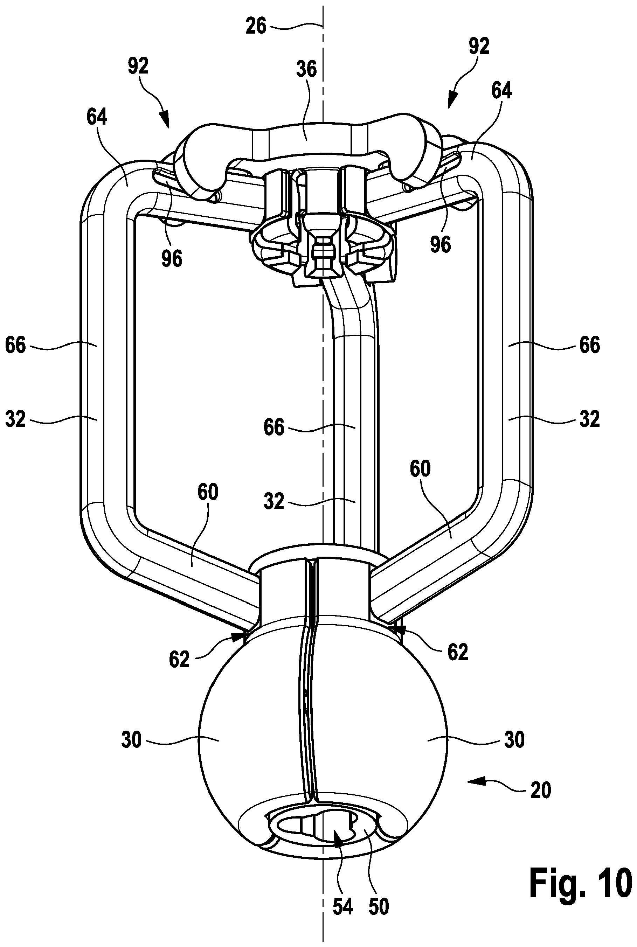

Fig. 10 zeigt den Spannring 36 aus Fig. 9, wobei nun die Armelemente 32 gezeigt sind, die in die Halterungen 92 eingehängt sind. Hierfür sind im Bereich der jeweiligen zweiten Armabschnitte 64 Nuten 96 an den Armelementen 32 ausgebildet. Bei diesem Aufbau sind die Armelemente 32 somit verschwenkbar am Spannring 36 angeordnet.

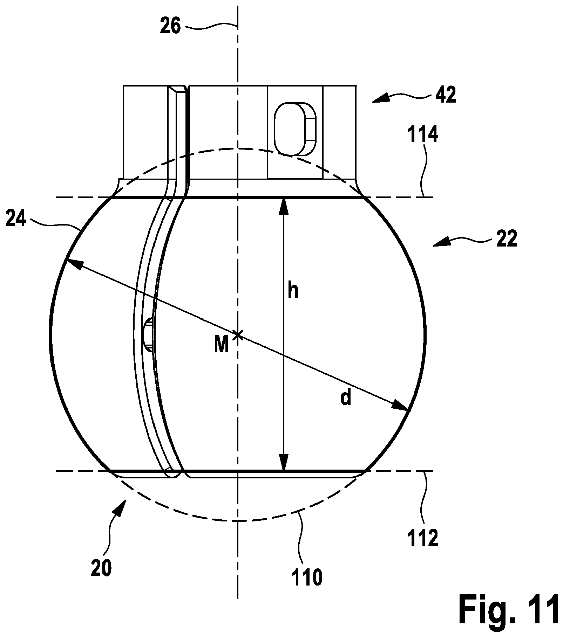

Fig. 11 erläutert die Form der Kugelscheibe 24, die den Halteabschnitt 22 aufweist. Dazu ist der Halteabschnitt 22 so dargestellt, dass die Längsachse 26 genau in der Zeichenebene verläuft. Die Kugelscheibe 24 entsteht, indem die Kugel 110 von den Ebenen 112 und 114 geschnitten wird, die senkrecht zur Längsachse 26 stehen. Die Kugelscheibe 24 hat einen Mittelpunkt M, einen Durchmesser d und eine Höhe h. Die Längsachse 26 der Vorrichtung ist dadurch definiert, dass sie durch den Mittelpunkt M und parallel zu der Höhe h verläuft.

Der Abstand der Ebenen 112 und 114 vom Mittelpunkt M entlang der Längsachse 26 ist hier zumindest ungefähr gleich. Die Abstände der Ebenen 112, 114 vom Mittelpunkt M können aber auch verschieden sein. Die Ebenen 112, 114 können ferner in einem Winkel zur Längsachse 26 stehen, sofern die Form einer Kugelscheibe weiterhin zumindest ungefähr gegeben ist.

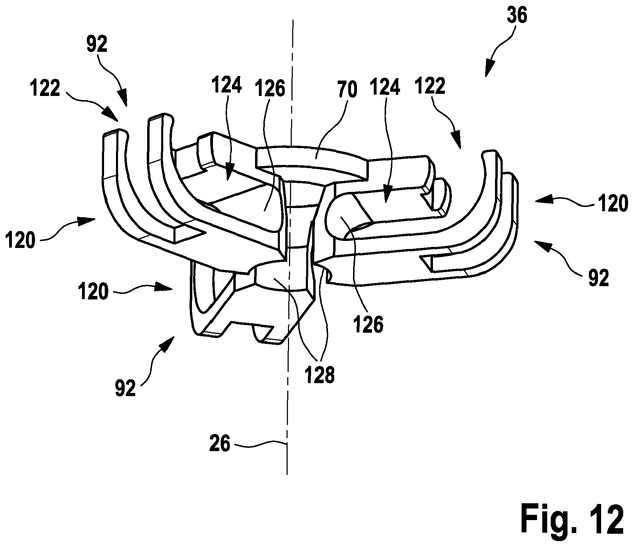

Fig. 12 zeigt eine dritte Ausführungsform eines Spannrings 36. Der Spannring 36 weist hier drei Halterungen 92 auf, die als Ausnehmungen 120 ausgebildet sind. Bezogen auf die Längsachse 26 beginnen die Ausnehmungen 120 hier mit einem außen gelegenen Einführungsabschnitt 122, der in einen Zuführungsabschnitt 124 übergeht.

Der Zuführungsabschnitt 124 ist durch eine Anlagefläche 126 begrenzt. Die Anlagefläche 126 ist dafür ausgebildet, einen Druck von der Auflagefläche 38 des jeweiligen Armelements 32 aufzunehmen, so dass eine korrespondierende und weiter innen liegende Druckfläche 128 in Richtung auf die Längsachse 26 hin gedrückt werden kann, insbesondere gegen das zweite Instrument 14.

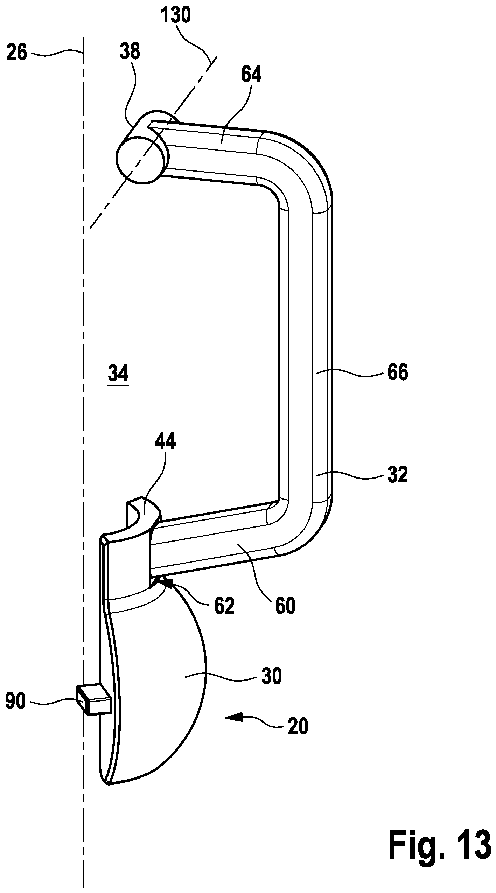

Fig. 13 zeigt ein Armelement 32 gemäß einer dritten Ausführungsform. Es gelten alle vorherigen Ausführungen zum Armelement 32. Die Auflagefläche 38 ist hier allerdings in der Form eines Zylinders ausgebildet. Es sind dabei auch andere gebogene Flächen denkbar, wie zum Beispiel eine halbzylindrische oder viertelzylindrische Fläche oder auch eine kugelförmige oder halbkugelförmige Fläche. Grundsätzlich können auch andere Formen der Auflagefläche 38 gewählt werden, beispielsweise wie in Fig. 5 gezeigt, sofern eine Kraftübertragung von dem Armelement 32 über die Auflagefläche 38, die Anlagefläche 126 auf die Druckfläche 128 gewährleistet ist. Eine gerundete Fläche wird im Hinblick auf eine einfache Verschwenkbarkeit der Armelemente 32 relativ zur Längsachse 26 als vorteilhaft angesehen. Grundsätzlich kann die Anlagefläche 38 aber beispielsweise auch durch einen Quader oder ein Prisma gebildet sein.

Bei der hier gezeigten Ausführungsform ist die Ausrichtung der zylinderförmigen Auflagefläche 38 so gewählt, dass die Mittelachse 130 der zylindrischen Auflagefläche 38 die Tangente eines Kreises darstellt, der senkrecht zur Längsachse 26 steht und die Längsachse 26 als Mittelpunkt hat.

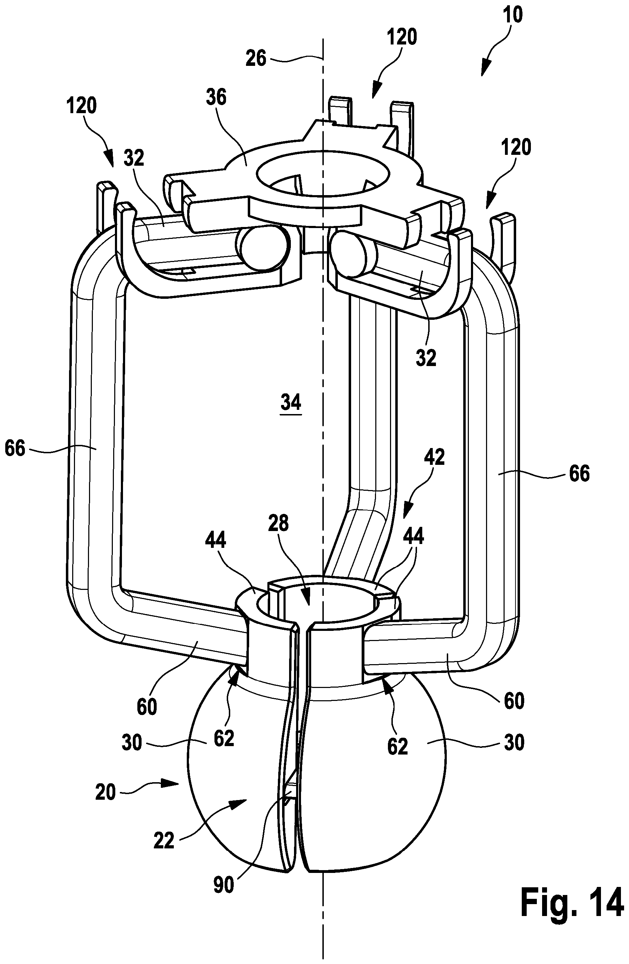

Fig. 14 zeigt eine dritte Ausführungsform der Vorrichtung 10, bei der Armelemente 32 gemäß Fig. 13 in den Spannring 36 gemäß Fig. 12 eingehängt sind.

## Patentansprüche

1. Vorrichtung (10) zur simultanen Fixierung von medizinischen Instrumenten (12, 14), die Vorrichtung (10) mit einem Gelenkteil (20), das einen Halteabschnitt (22) in der Form einer Kugelscheibe (24) aufweist, wobei die Kugelscheibe (24) einen Mittelpunkt (M) und eine Höhe (h) aufweist und eine Längsachse (26) der Vorrichtung (10) definiert, die durch den Mittelpunkt (M) und parallel zu der Höhe (h) verläuft, wobei die Kugelscheibe (24) entlang der Längsachse (26) einen durchgängigen Hohlraum (28) aufweist, wobei der Halteabschnitt (22) in mindestens zwei Haltesegmente (30) unterteilt ist und wobei an dem Gelenkteil (20) mindestens zwei Armelemente (32) angeordnet sind, die jeweils in einem ersten Armabschnitt (60) ausgehend von einem Befestigungspunkt (62) am Gelenkteil (20) sich von der Längsachse (26) entfernen, in einem zweiten Armabschnitt (64) sich der Längsachse (26) annähern und mit einer Auflagefläche (38) enden, wodurch ein Freiraum (34) zwischen den Armelementen (32) gebildet ist, die Vorrichtung (10) ferner mit einem Spannelement (40), welches dafür ausgebildet ist, die Haltesegmente (30) zusammenzudrücken, wobei bei einem Zusammendrücken der Haltesegmente (30) sich die Auflageflächen (38) auf die Längsachse (26) zu bewegen.

2. Vorrichtung nach Anspruch 1, wobei das Gelenkteil (20) einen Flansch (42) aufweist, der sich ausgehend von dem Halteabschnitt (22) entlang der Längsachse (26) erstreckt und eine Fortsetzung des Hohlraums (28) bildet, wobei der Flansch (42) in mindestens zwei Flanschsegmente (44) unterteilt ist, die insbesondere jeweils mit genau einem Haltesegment (30) verbunden sind.

3. Vorrichtung nach einem der vorhergehenden Ansprüche, wobei die Kugelscheibe (24) einen Durchmesser (d) aufweist und das Verhältnis von der Höhe (h) zu dem Durchmesser (d) zwischen 0,35 und 0,99 liegt, bevorzugt zwischen 0,47 und 0,93 liegt, besonders bevorzugt zwischen 0,58 und 0,87 liegt und insbesondere zwischen 0,7 und 0,8 liegt.

4. Vorrichtung nach einem der vorhergehenden Ansprüche ferner mit einem Spannring, der von den Auflageflächen (38) gehalten ist, wobei bei einem Zusammendrücken der Haltesegmente (30) die Auflageflächen (38) gegen den Spannring (36) drücken.

5. Vorrichtung nach Anspruch 4, wobei die Armelemente (32) verschwenkbar am Spannring (36) angeordnet sind und/oder die Auflageflächen (38) gebogen sind, um der Form des Spannrings (36) zu folgen.

6. Vorrichtung nach Anspruch 4 oder 5, wobei der Spannring (36) für jede Auflagefläche (38) eine Aufnehmung (120) aufweist, in die eine jeweilige Auflagefläche (38) lösbar eingehängt werden kann, so dass das jeweilige Armelement (32) um die jeweilige Auflagefläche (38) als Schwenkpunkt relativ zur Längsachse (26) verschwenkt werden kann.

7. Vorrichtung nach einem der vorhergehenden Ansprüche, wobei zwei benachbarte Haltesegmente (30) über eine Nut-Feder-Verbindung (82) in Eingriff miteinander stehen.

8. Vorrichtung nach einem der vorhergehenden Ansprüche, wobei der Halteabschnitt (22) genau drei Haltesegmente (30) aufweist und an jedem Haltesegment (30) genau ein Armelement (32) angeordnet ist.

9. Vorrichtung nach einem der vorhergehenden Ansprüche, wobei die Armelemente (32) bezogen auf einen Winkel (a) um die Längsachse (26) um mindestens 45°, bevorzugt um mindestens 60°, besonders bevorzugt um mindestens 90° und insbesondere um mindestens 120° voneinander beabstandet sind.

10. Vorrichtung nach einem der vorhergehenden Ansprüche, wobei jedes Armelement (32) einen dritten Armabschnitt (66) aufweist, der zwischen dem ersten Armabschnitt (60) und dem zweiten Armabschnitt (64) angeordnet ist und sich insbesondere zumindest ungefähr parallel zu der Längsachse (26) erstreckt.

11. Vorrichtung nach einem der vorhergehenden Ansprüche, wobei das Spannelement (40) zwei Spannhebel (74) aufweist, die mit einem Scharnier (76) verbunden sind, wobei jeder Spannhebel (74) einen Druckbereich (78) aufweist, der dafür ausgebildet ist, das Gelenkteil (20) zu halten und gegen den Halteabschnitt (22) zu drücken, wenn die Spannhebel (74) zusammengedrückt werden, wobei die Spannhebel (74) insbesondere mit einer Spannschraube (80) verbunden sind, die dafür ausgebildet ist, die Spannhebel (74) zusammenzudrücken.

12. Vorrichtung nach einem der Ansprüche 4 bis 10, wobei der Spannring (36) ein ringförmiges Basiselement (70) und mindestens zwei Fortsätze (72) aufweist, die relativ zum Basiselement (70) auf die Längsachse (26) zu verlagerbar sind und wobei der Spannring (36) an den Fortsätzen (72) gehalten ist.

13. Vorrichtung nach einem der vorhergehenden Ansprüche, wobei in den Halteabschnitt ein elastisches Halteelement (50) eingesetzt ist, das dafür ausgebildet ist, ein erstes Instrument (12) der medizinischen Instrumente (12, 14) entlang der Längsachse (26) aufzunehmen.

14. Vorrichtung nach einem der vorhergehenden Ansprüche, wobei der Halteabschnitt (22) dafür ausgebildet ist, ein erstes Instrument (12) der medizinischen Instrumente (12, 14), insbesondere einen Trokar, zu führen, und die Auflageflächen (38) dafür ausgebildet ist, ein zweites Instrument (14) medizinischen Instrumente (12, 14), insbesondere ein in den Trokar eingeführtes Endoskop, zu halten.

15. System (104) mit einer Vorrichtung (10) nach einem der vorhergehenden Ansprüche, einem Trokar und einem Endoskop, wobei der Trokar von dem Halteabschnitt (22) entlang der Längsachse gehalten ist und das Endoskop in den Trokar eingeführt ist und von dem Spannring (36) entlang der Längsachse (26) gehalten ist.

## Claims

1. A device (10) for simultaneous fixation of medical instruments (12, 14), the device (10) comprising a joint element (20) having a holding portion (22) in the form of a spherical disk (24), the spherical disc (24) having a center point (M) and a height (h) and defining a longitudinal axis (26) of the device (10), which extends through the center point (M) and parallel to the height (h), wherein the spherical disc (24) has a continuous cavity (28) along the longitudinal axis (26), wherein the holding portion (22) is divided into at least two holding segments (30) and wherein at least two arm elements (32) are arranged on the joint element (20), which each veer away from the longitudinal axis (26) in a first arm section (60) starting from an attachment point (62) on the joint element (20), approach the longitudinal axis (26) in a second arm section (64) and end with a bearing surface (38), whereby a free space (34) is formed between the arm elements (32), the device (10) further comprising a clamping element (40) which is adapted to compress the holding segments (30), wherein, when the holding segments (30) are pushed together, the bearing surfaces (38) move towards the longitudinal axis (26).

2. The device according to claim 1, wherein the joint element (20) has a flange (42) which, starting from the holding portion (22), extends along the longitudinal axis (26) and forms a continuation of the cavity (28), wherein the flange (42) is divided into at least two flange segments (44) which, in particular, are each connected to exactly one holding segment (30).

3. The device according to one of the preceding claims, the spherical disc (24) having a diameter (d), and the ratio of the height (h) to the diameter (d) being between 0.35 and 0.99, preferably between 0.47 and 0.93, particularly preferably between 0.58 and 0.87, and particularly between 0.7 and 0.8.

4. The device according to one of the preceding claims further comprising a clamping ring which is held by the bearing surfaces (38), wherein when the holding segments (30) are pushed together, the bearing surfaces (38) press against the clamping ring (36).

5. The device according to claim 4, wherein the arm elements (32) are pivotally arranged on the clamping ring (36) and/or the bearing surfaces (38) are bent to follow the shape of the clamping ring (36).

6. The device according to claim 4 or 5, wherein the clamping ring (36) has a recess (120) for each bearing surface (38), into which a respective bearing surface (38) can be detachably suspended so that the respective arm element (32) can be pivoted about the respective bearing surface (38) as a pivot point relative to the longitudinal axis (26).

7. The device according to one of the preceding claims, wherein two adjacent holding segments (30) are in engagement with each other via a tongue-and-groove connection (82).

8. The device according to one of the preceding claims, wherein the holding portion (22) has exactly three holding segments (30) and exactly one arm element (32) is arranged on each holding segment (30).

9. The device according to one of the preceding claims, wherein the arm elements (32) are spaced apart from one another by at least 45°, preferably by at least 60°, particularly preferably by at least 90° and in particular by at least 120° with respect to an angle (a) about the longitudinal axis (26).

10. The device according to one of the preceding claims, each arm element (32) having a third arm portion (66) which is arranged between the first arm portion (60) and the second arm portion (64) and, in particular, extends at least approximately parallel to the longitudinal axis (26).

11. The device according to one of the preceding claims, the clamping element (40) comprising two clamping levers (74) connected to a hinge (76), each clamping lever (74) having a pressure portion (78) adapted to hold the joint element (20) and to press it against the holding portion (22) when the clamping levers (74) are compressed, the clamping levers (74) being connected, in particular, to a clamping screw (80) adapted to compress the clamping levers (74).

12. The device according to one of claims 4 to 10, wherein the clamping ring (36) comprises an annular base element (70) and at least two extensions (72) which are displaceable relative to the base element (70) towards the longitudinal axis (26) and wherein the clamping ring (36) is held at the extensions (72).

13. The device according to one of the preceding claims, wherein an elastic holding element (50) is inserted into the holding portion, which is adapted to receive a first instrument (12) of the medical instruments (12, 14) along the longitudinal axis (26).

14. The device according to one of the preceding claims, wherein the holding portion (22) is adapted to guide a first instrument (12) of the medical instruments (12, 14), in particular a trocar, and the supporting surfaces (38) are adapted to hold a second instrument (14) of the medical instruments (12, 14), in particular an endoscope inserted into the trocar.

15. A system (104) comprising a device (10) according to any of the foregoing claims, a trocar and an endoscope, the trocar being supported by the holding portion (22) along the longitudinal axis and the endoscope being inserted into the trocar and supported by the clamping ring (36) along the longitudinal axis (26).

## Revendications

1. Dispositif (10) pour la fixation simultanée d'instruments médicaux (12, 14), le dispositif (10) comprenant une partie d'articulation (20) qui présente une portion de retenue (22) en forme de rondelle sphérique (24), la rondelle sphérique (24) présentant un centre (M) et une hauteur (h) et définissant un axe longitudinal (26) du dispositif (10) qui s'étend à travers le centre (M) et parallèlement à la hauteur (h), la rondelle sphérique (24) présentant le long de l'axe longitudinal (26) une cavité continue (28), la portion de retenue (22) étant divisée en au moins deux segments de retenue (30) et au moins deux éléments de bras (32) étant disposés au niveau de la partie d'articulation (20), lesquels, dans une première portion de bras (60), à partir d'un point de fixation (62) au niveau de la partie d'articulation (20), s'éloignent de l'axe longitudinal (26), et, dans une deuxième portion de bras (64), se rapprochent de l'axe longitudinal (26) et se terminent par une surface d'appui (38), de sorte qu'un espace libre (34) soit formé entre les éléments de bras (32), le dispositif (10) comprenant en outre un élément de serrage (40) qui est réalisé de manière à comprimer les segments de retenue (30), les surfaces d'appui (38) se déplaçant vers l'axe longitudinal (26) lors d'une compression des segments de retenue (30).

2. Dispositif selon la revendication 1, dans lequel la partie d'articulation (20) présente une bride (42) qui s'étend à partir de la portion de retenue (22) le long de l'axe longitudinal (26) et forme un prolongement de la cavité (28), la bride (42) étant divisée en au moins deux segments de bride (44) qui sont notamment à chaque fois raccordés à exactement un segment de retenue (30).

3. Dispositif selon l'une quelconque des revendications précédentes, dans lequel la rondelle sphérique (24) présente un diamètre (d) et le rapport de la hauteur (h) au diamètre (d) est compris entre 0,35 et 0,99, de préférence entre 0,47 et 0,93, particulièrement préférablement entre 0,58 et 0,87 et en particulier entre 0,7 et 0,8.

4. Dispositif selon l'une quelconque des revendications précédentes, comprenant en outre une bague de serrage qui est retenue par les surfaces d'appui (38), les surfaces d'appui (38) pressant contre la bague de serrage (36) lors d'une compression des segments de retenue (30).

5. Dispositif selon la revendication 4, dans lequel les éléments de bras (32) sont disposés de manière pivotante sur la bague de serrage (36) et/ou les surfaces d'appui (38) sont cintrées afin de suivre la forme de la bague de serrage (36).

6. Dispositif selon la revendication 4 ou 5, dans lequel la bague de serrage (36) présente pour chaque surface d'appui (38) un logement (120) dans lequel peut être accrochée de manière détachable une surface d'appui respective (38) de telle sorte que l'élément de bras respectif (32) puisse être pivoté autour de la surface d'appui respective (38) en tant que pivot par rapport à l'axe longitudinal (26).

7. Dispositif selon l'une quelconque des revendications précédentes, dans lequel deux segments de retenue adjacents (30) sont en prise l'un avec l'autre par le biais d'une liaison à rainure et languette (82).

8. Dispositif selon l'une quelconque des revendications précédentes, dans lequel la portion de retenue (22) présente exactement trois segments de retenue (30) et exactement un élément de bras (32) est disposé au niveau de chaque segment de retenue (30).

9. Dispositif selon l'une quelconque des revendications précédentes, dans lequel les éléments de bras (32) sont espacés l'un de l'autre par rapport à un angle (α) autour de l'axe longitudinal (26) d'au moins 45°, de préférence d'au moins 60°, particulièrement préférablement d'au moins 90° et en particulier d'au moins 120°.

10. Dispositif selon l'une quelconque des revendications précédentes, dans lequel chaque élément de bras (32) présente une troisième portion de bras (66) qui est disposée entre la première portion de bras (60) et la deuxième portion de bras (64) et qui s'étend en particulier au moins approximativement parallèlement à l'axe longitudinal (26).

11. Dispositif selon l'une quelconque des revendications précédentes, dans lequel l'élément de serrage (40) présente deux leviers de serrage (74) qui sont reliés par une charnière (76), chaque levier de serrage (74) présentant une région de pression (78) qui est réalisée de manière à retenir la partie d'articulation (20) et à la presser contre la portion de retenue (22) lorsque les leviers de serrage (74) sont comprimés, les leviers de serrage (74) étant notamment reliés par une vis de serrage (80) qui est réalisée de manière à comprimer les leviers de serrage (74).

12. Dispositif selon l'une quelconque des revendications 4 à 10, dans lequel la bague de serrage (36) présente un élément de base de forme annulaire (70) et au moins deux saillies (72) qui peuvent être déplacées par rapport à l'élément de base (70) sur l'axe longitudinal (26) et la bague de serrage (36) étant retenue au niveau des saillies (72).

13. Dispositif selon l'une quelconque des revendications précédentes, dans lequel un élément de retenue élastique (50) est inséré dans la portion de retenue, lequel est réalisé de manière à recevoir un premier instrument (12) parmi les instruments médicaux (12, 14) le long de l'axe longitudinal (26).

14. Dispositif selon l'une quelconque des revendications précédentes, dans lequel la portion de retenue (22) est réalisée de manière à guider un premier instrument (12) parmi les instruments médicaux (12, 14), en particulier un trocart, et les surfaces d'appui (38) sont réalisées de manière à retenir un deuxième instrument (14) des instruments médicaux (12, 14), en particulier un endoscope introduit dans le trocart.

15. Système (104) comprenant un dispositif (10) selon l'une quelconque des revendications précédentes, un trocart et un endoscope, le trocart étant retenu par la portion de retenue (22) le long de l'axe longitudinal et l'endoscope étant introduit dans le trocart et étant retenu par la bague de serrage (36) le long de l'axe longitudinal (26).
